(19) Europäisches Patentamt
European Patent Office
Office européen des brevets

(11) EP 0 258 676 B1

(12) # EUROPÄISCHE PATENTSCHRIFT

(45) Veröffentlichungstag und Bekanntmachung des Hinweises auf die Patenterteilung:
**27.12.1996 Patentblatt 1996/52**

(51) Int Cl.[6]: **C07D 251/52**, C08K 5/37

(21) Anmeldenummer: **87111430.2**

(22) Anmeldetag: **07.08.1987**

(54) **Verfahren zur Herstellung von Mischungen schwefelhaltiger Triazinverbindungen**

Process for the preparation of mixtures of sulphur-containing triazine compounds

Procédé de préparation de mélanges de composés de triazine soufrés

(84) Benannte Vertragsstaaten:
**AT BE CH DE FR GB IT LI LU NL SE**

(30) Priorität: **04.09.1986 DE 3630055**

(43) Veröffentlichungstag der Anmeldung:
**09.03.1988 Patentblatt 1988/10**

(73) Patentinhaber: **Thomas Swan and Co. Ltd**
**Consett, County Durham DH6 7ND (GB)**

(72) Erfinder:
- **Deschler, Ulrich, Dr.**
**D-6450 Hanau 9 (DE)**
- **Hellwig, Georg, Dr.**
**D-6466 Gründau 2 (DE)**
- **Michel, Rudolf**
**D-6463 Freigericht (DE)**
- **Kleinschmit, Peter, Dr.**
**D-6450 Hanau 9 (DE)**
- **Wolff, Siegfried**
**D-5303 Bornheim (DE)**

(74) Vertreter: **Benedum, Ulrich Max, Dr. et al**
**Haseltine Lake Partners**
**Motorama Haus 502**
**Rosenheimer Strasse 30**
**81669 München (DE)**

(56) Entgegenhaltungen:
**EP-A- 239 816**
**EP-A- 0 038 459**
**EP-A- 0 178 444**
**FR-A- 2 099 583**
**US-A- 3 923 724**

- **ORGANIC CHEMISTRY OF SULFUR (1977), OAE S. ED., PLENUM PRESS, SEITE 328, TEIL 7.3.4**
- **MECHANISM IN ORGANIC CHEMISTRY (1971), ALDER R.W. ET AL., WILEY INTERSCIENCE, "NUCLEOPHILIC AROMATIC SUBSTITUTION", SEITEN 342-343**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft ein Verfahren zur Herstellung von Mischungen schwefelhaltiger Triazinverbindungen.

Aus der DE-PS 1 699 954 ist das Bis-(2-ethylamino-4-diethylamino-s-triazin-6-yl)disulfan bekannt. Man kann es beispielsweise aus dem entsprechenden Monomercaptotriazin durch Oxidation mit Jod oder Wasserstoffperoxid herstellen. Die so gewonnene Verbindung wird als Vulkanisationsbeschleuniger in Kautschukmischungen eingesetzt.

Das entsprechende Tetrasulfan wird in der DE-OS 34 38 290 beschrieben.

Diese Verbindung entsteht bei der Umsetzung von Mercaptotriazinen mit $S_2Cl_2$ und wird mit Erfolg in vulkanisierbaren Mischungen als Vernetzer bzw. als Vulkanisationsbeschleuniger eingesetzt.

Dieselbe Wirkung entfalten gemäß EP-A-0 239 816 solche Mischungen aus Bistriazinylpolysulfanen, bei denen nicht eine definierte $S_4$-Brücke die beiden Triazinringe verbindet, sondern eine Polysulfankette mit einer statistisch zusammengesetzten Länge von vier Schwefelatomen.

Gegenstand der Erfindung ist ein Verfahren zur Herstellung von Mischungen schwefelhaltiger Triazinverbindungen der allgemeinen Formel

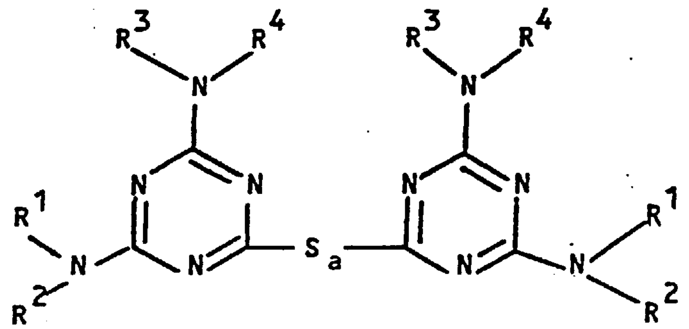

(I)

in welcher bedeuten:

| | |
|---|---|
| $R^1$, $R^2$ = | H, $R^2$ = Benzyl, |
| $R^2$, $R^3$, $R^4$ = | $C_1$-$C_8$ -Alkyl, Allyl, $C_3$-$C_8$ Cycloalkyl, letzteres unsubstituiert oder mit 1-3 Methylgruppen substituiert, 2-Hydroxiethyl, 3-Hydroxipropyl, 2-Hydroxipropyl oder |
| $R^3$ und $R^4$ (zusammen): | $C_4$-$C_6$-Alkylen,-$(CH_2$-$CHX)_2$ Y mit X = H, $CH_3$, Y = O,S, |
| | $S_a$: Polysulfankette mit 2-10 S-Atomen (d.h. $2 \leq a \leq 10$), wobei die einzelnen Polysulfane in solchen Konzentrationen vorliegen, daß das statistische Mittel $\bar{a}$ ganze oder gebrochene Zahlenwerte von 2 bis 5 annimmt, dadurch gekennzeichnet, daß man eine Triazinverbindung der allgemeinen Formel |

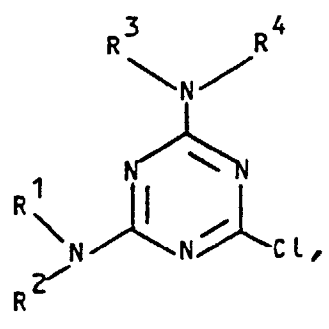

in der $R^1$, $R^2$,$R^3$, $R^4$ die oben angegebenen Bedeutungen besitzen,

mit einer in einem protischen, polaren, Lösungsmittel, insbesondere Wasser oder deren Gemischen mit Wasser gelösten Verbindung der Formel

$$Me_2S_{\bar{a}} \quad \text{(III)},$$

in der Me das Ammonium- oder ein Alkalikation, bevorzugt $Na^+$ oder $K^+$ bedeutet, und $\bar{a}$ dem statistischen Mittelwert mit $2 \leq \bar{a} \leq 5$ entspricht, im Molverhältnis 2:1 bis 2:1,1 bei einer Temperatur von 80 bis 140°C umsetzt und aus dem anfallenden Reaktionsgemisch das Produkt in an sich bekannter Weise abtrennt.
Bevorzugt läßt man die Reaktion bei der sich einstellenden Rückflußtemperatur ablaufen; bildet Wasser die Hauptkomponente des Lösungsmittels insbesondere bei 95 - 100°C. Es ist jedoch auch möglich, bei höheren Temperaturen und wegen des sich einstellenden Drucks die Umsetzung in einem Druckbehälter vorzunehmen.

In einer bevorzugten Ausführungsform tropft man die geschmolzene Verbindung gemäß Formel II in die stark alkalische, wässrige Lösung des $Me_2S_{\bar{a}}$, die auf 100°C erhitzt ist.

In wasserhaltigen Reaktionsgemischen sinkt während der Umsetzung der pH-Wert ab. In einer bevorzugten Ausführungsform dosiert man daher, insbesondere wenn Wasser den überwiegenden Teil des Lösungsmittels oder dessen Gesamtmenge ausmacht, eine wäßrige alkalische Lösung, beispielsweise Natronlauge oder Natriumbicarbonatlösung, während der Reaktion kontinuierlich zu, so daß der pH-Wert nicht unter 9,0 bis 7,5, insbesondere bis 8,5 sinkt.

Das eingesetzte $Me_2S_{\bar{a}}$ ist bezüglich der Polysulfidkettenlänge statistisch zusammengesetzt. Der stöchiometrische Faktor 4 in $Na_2S_4$ beispielsweise besagt daher lediglich, daß die im Gemisch vorhandenen Dianionen $S_a^{2\ominus}$ mit $2 \leq a \leq 10$ in solchen gegenseitigen Verhältnissen vorliegen, daß die folgende Beziehung erfüllt ist:

$$\frac{\Sigma n_a \cdot a}{n} = 4 = \bar{a}$$

mit

n = Gesamtzahl vorhandener Dianionen
$n_a$ = Zahl vorhandener Dianionen der Kettenlänge a.
$\bar{a}$ = statistischer Mittelwert

Da alle in $Me_2S_{\bar{a}}$ vorliegenden Dianionen in chemischen Umsetzungen nukleophile Eigenschaften aufweisen, ist bei der erfindungsgemäßen Umsetzung von vorneherein mit einem bezüglich der Polysulfankettenlängen statistisch zusammengesetztem Produktgemisch zu rechnen.

Nach spätestens 3 Stunden bei 100°C in wässrigem Medium ist die Reaktion abgeschlossen. Zur Sicherheit kann das Reaktionsgemisch aber auch länger unter Rückfluß gekocht werden.

Anschließend läßt man das sich als untere Phase absetzende schwefelhaltige Triazinderivat in der Hitze flüssig ablaufen, läßt es dann abkühlen und entfernt Feuchtigkeitsreste durch Trocknung zum Beispiel im Vakuum.

Die Überführung in die für die Verwendung in Kautschukmischungen notwendige feinteilige Form wird nach dem Stand der Technik zum Beispiel durch Zermahlen vorgenommen.

Sollte die Analyse auf freien Schwefel in dem Endprodukt hindeuten, kann eine Reinigung beispielsweise durch Lösen des Rohproduktes in heißem Ethanol erfolgen.

Danach lassen sich Verunreinigungen wie Schwefel oder auch NaCl durch Filtration abtrennen.

Eine Herstellungsvariante besteht darin, das $Me_2S_{\bar{a}}$ zum Beispiel in Ethanol gelöst vorzulegen oder als Lösungsmittel n-Propanol oder Glykol zu verwenden, ebenso wie Mischungen der genannten Alkohole mit Wasser.

Das Triazinderivat gemäß Formel (II) wird dann in Pulverform oder bevorzugt in dem entsprechenden Alkohol gelöst bzw. suspendiert zudosiert oder in einer anderen Variante vorgelegt und anschließend mit der $Me_2S_a$-Lösung versetzt. Zur Produktabtrennung kann das Reaktionsgemisch dann zum Beispiel mit Wasser versetzt und abfiltriert werden. Der unlösliche Filterrückstand wird dann getrocknet und zermahlen.

Als Beispiel bevorzugter nach dem erfindungsgemäßen Verfahren herstellbaren Mischungen seien die folgenden Produkte genannt:

A Bis-(2-ethylamino-4-di-isopropylamino-s-triazin-6-yl)-oligosulfid

B Bis-(2-n-butylamino-4-diethylamino-s-triazin-6-yl)-oligo-sulfid

C Bis-(2-isopropylamino-4-di-isopropylamino-s-triazin-6-yl)oligosulfid

D Bis-(2-ethylamino-4-di-isobutylamino-s-triazin-6-yl)-oligosulfid

E Bis-(2-ethylamino-4-di-n-propylamino-s-triazin-6-yl)-oligosulfid

F Bis-(2-n-propylamino-4-diethylamino-s-triazin-6-yl)-oligosulfid

G Bis-(2-n-propylamino-4-di-n-propylamino-s-triazin-6-yl-)-oligosulfid

H Bis-(2-n-butylamino-4-di-n-propylamino-s-triazin-6-yl)-oligosulfid

I Bis-(2-ethylamino-4-di-n-butylamino-s-triazin-6-yl)-oligosulfid

K Bis-(2-cyclohexylamino-4-diethylamino-s-triazin-6-yl)-oligosulfid

L Bis-(2-ethylamino-4-diethylamino-s-triazin-6-yl)-oligosulfid

M Bis-(2-amino-4-diethylamino-s- triazin-6-yl)-oligosulfid

Als Oligosulfide sind dabei die Verbindungen zu verstehen, bei denen die einzelnen Polysulfane mit einer S-Kettenlänge $2 \leq a \leq 10$ in solchen Verhältnissen vorliegen, daß der statistische Mittelwert der S-Kettenlänge $\bar{a}$ ganze oder gebrochene Zahlenwerte von 2 bis 5 annimmt ($2 \leq \bar{a} \leq 5$).

Beispiel 1:

Man legt in dieser Reihenfolge 229.7 g entsprechend 1.0 Mol 2-Ethylamino-4-diethylamino-6-chlortriazin, 95,8 g entsprechend 0,55 Mol eines Dinatriumpolysulfidgemisches der statistischen Zusammensetzung $Na_2S_4$ (Herstellung vgl. DE-OS 34 36 698) sowie 1000 ml $H_2O$ in einem 21-Dreihalskolben mit KPG-Rührer, Rückflußkühler und pH-Elektrode vor und heizt dieses Reaktionsgemisch innerhalb von 30 min auf Rückflußtemperatur auf. Dabei sinkt der anfänglich gemessene pH-Wert von 10,7 allmählich ab und erreicht nach 2 h Refluxierdauer einen Wert von pH=8,5. Parallel dazu wird eine Farbänderung des Reaktionsmediums von tieforange-braun nach gelb beobachtet. Es wird nun für eine weitere Stunde unter Rückfluß gerührt, wobei durch Zudosierung einer wässrigen NaOH-Lösung (20%ig) der pH-Wert auf ≥ 8,5 gehalten wird (Verbrauch: 0,1 Mol Na0H). Nach Abstellen des KPG-Rührers bildet sich ein 2-Phasensystem aus, wobei der im wesentlichen aus dem Produktgemisch bestehende, spezifisch schwerere Anteil über ein Bodenablaßventil von der wässrigen Phase abgetrennt wird und zu einem gelbbraunen, Bernstein-artigen Feststoff erstarrt (Erstarrungspunkt ca. 90°C). Die ca. 5% darin enthaltenen Feuchtigkeitsreste werden durch Vakuumbehandlung (15 Torr $\triangleq 20 \cdot 10^2$Pa) bei 95°C für die Dauer von 16 h bis auf eine Restfeuchte von ≤ 0,5% entfernt. Der so erhaltene Feststoff läßt sich im Mörser zu einem bei Raumtemperatur rieselfähigen Pulver zerkleinern. Dieses Pulver ist in Ethanol klar löslich, d.h., es enthält keine signifikanten Mengen an elementarem Schwefel oder an NaCl

Ausbeute: 246,5 g entsprechen 95,4 % d.Th., hellgelbes Pulver

| Elementenanalyse: $C_{18}H_{32}N_{10}S_4$ (516,76) | | | | | |
|---|---|---|---|---|---|
| | C | H | N | S | Cl |
| ber.: | 41,84 | 6,24 | 27,10 | 24,82 | 0 |
| gef.: | 41.07 | 6,24 | 26,68 | 24,80 | 0,1 |

HPLC-Analyse (nach bekannter Methode, K.0.Hiller et al; Z.Anal.Chem. 280 (1976), 293):

| Verbindung *) | rel. Flächen-% **) |
|---|---|
| $R-S_2-R$ | 48,5 |
| $R-S_3-R$ | 12,6 |
| $R-S_4-R$ | 18,8 |
| $R-S_5-R$ | 8,8 |
| $R-S_6-R$ | 6,7 |

*) R = 2-Ethylamino-4-diethylamino-6-triazinyl

**) unter Vernachlässigung der durch das Laufmittel verursachten peak-Flächen, Normierung auf 100 Fl.-%

(fortgesetzt)

| Verbindung *) | rel. Flächen-% **) |
|---|---|
| R-$S_7$-R | 3,1 |
| R-$S_8$-R | 1,5 |

*) R = 2-Ethylamino-4-diethylamino-6-triazinyl

**) unter Vernachlässigung der durch das Laufmittel verursachten peak-Flächen, Normierung auf 100 Fl.-%

Beispiel 2:

In einer zu der in Beispiel 1 beschriebenen analogen Standardapparatur werden 25,5 g entsprechend 0,13 Mol Diammoniumpentasulfid (Herstellung vgl. J.S. Thomas et al., J.Chem. Soc. 1923, 1726 ff) in 300 ml Wasser gelöst. Zur entstehenden rotbraunen Ammoniumpolysulfidlösung wurden bei Rückflußtemperatur 57,4 g entsprechend 0,25 Mol verflüssigtes 2-Ethylamino-4-diethylamino-6-chlortriazin (Schmelzpunkt: 98°C) innerhalb von 30 min aus einem beheizten Tropftrichter unter Rühren zudosiert. Die Reaktionslösung wird für weitere 90 min unter Rückfluß gerührt, wobei sich der anfangs gemessene pH-Wert von 11,7 langsam auf 8,0 absenkt. Durch Zudosierung einer wässrigen NaOH-Lösung wird der pH-Wert für die folgenden 60 min auf 9,0 gehalten (Verbrauch: 0,04 Mol NaOH). Wie in Beispiel 1 beschrieben wird das Produktgemisch über ein Bodenablaßventil isoliert, getrocknet und gepulvert. Es werden 66,3 g eines gelblichen Pulvers erhalten, das in heißem Ethanol nicht vollständig löslich ist. Nach Behandlung im Soxhlet-Extraktor mit 150 ml Ethanol bleiben 2,6 g eines gelben Pulvers mit einem S-Gehalt von 98,6 % zurück. Das Produkt wird aus dem Extrakt durch Abziehen des Lösungsmittel isoliert

Ausbeute: 63,2 g entsprechend 92,1 % d.Th. (bezogen auf ein statistisch zusammengesetztes Pentasulfan), hellgelbes Pulver

| Elementenanalyse: $C_{18}$ $H_{32}$ $N_{10}$ $S_{4,5}$ (532,79) | | | | | |
|---|---|---|---|---|---|
| | C | H | N | S | Cl |
| ber.: | 40,58 | 6,05 | 26,29 | 27,08 | 0 |
| gef.: | 41,12 | 6,43 | 27,12 | 26,61 | 0,2 |

HPLC-Analyse

| Zuordnung | rel. Flächen-% |
|---|---|
| R-$S_2$-R | 9,0 |
| R-$S_3$-R | 29,8 |
| R-$S_4$-R | 37,8 |
| R-$S_5$-R | 12,4 |
| R-$S_6$-R | 6,5 |
| R-$S_7$-R | 3,1 |
| R-$S_8$-R | 1,4 |

Beispiel 3:

Das in Beispiel 1 beschriebene Verfahren wird mit den folgenden Edukten durchgeführt:

71,4 g entsprechend 0,25 Mol 2-Ethylamino-4-di-n-butylamino-6-chlortriazin;

22,7 g Dikaliumtrisulfid (Herstellung vgl. Brauer, Bd. 1, S. 375);

250 ml Wasser;

Es werden 71,8 g eines hellgelben Feststoffes entsprechend 96,3 % d.M. isoliert mit den folgenden analytischen Daten:

| $C_{26}H_{48}N_{10}S_3$ (596,93): | | | | | |
|---|---|---|---|---|---|
| | C | H | N | S | Cl |
| ber.: | 52,31 | 8,11 | 23,46 | 16,11 | 0 |
| gef.: | 52,67 | 8,62 | 23,83 | 15,89 | 0,15 |

Das HPLC-Diagramm enthält Signale für das Bistriazinyldisulfan (45,7 %-Fl.), das entsprechende Tri- (39,1 %), Tetra- (12,8 %) und Pentasulfan (2,4 %).

Beispiel 4:

Das in Beispiel 1 beschriebene Verfahren wird mit den folgenden Edukten durchgeführt:

71 g entsprechend 0,25 Mol 2-Cyclohexylamino-4-diethylamino-6-chlortriazin;

13,8 g entsprechend 0,13 Mol Dinatriumdisulfid (Herstellung vgl. Brauer, Bd. 1, S. 374);

300 ml Wasser;

Es werden 54,3 g eines hellgelben Feststoffes entsprechend 87,4 % d.M. isoliert mit den folgenden analytischen Daten:

| $C_{26}H_{44}N_{10}S_2$ (560,84): | | | | | |
|---|---|---|---|---|---|
| | C | H | N | S | Cl |
| ber.: | 55,68 | 7,91 | 24,97 | 11,43 | 0 |
| gef.: | 56,21 | 7,98 | 25,31 | 10,73 | 0,4 |

Das HPLC-Diagramm enthält Signale für das Bistriazinyldisulfan (89,1 Fl.-%) neben denen für das entsprechende Tri- (9,7 %) und Tetrasulfan (1,2 %);

Beispiele 5-9:

Das in Beispiel 2 beschriebene Verfahren (Zudosierung von 2-Ethylamino-4-diethylamino-6-chlortriazin in flüssiger Form) wurde unter Verwendung von Dinatriumpolysulfanen (Herstellung aus den Elementen gemäß DE-OS 34 36 698) in verschiedenen Lösungsmitteln durchgeführt. Reaktions- und Produktdaten sind in den Tabellen 1 und 2 aufgelistet. Zur Produktaufarbeitung wurde das Reaktionsgemisch jeweils so lange bei 95°C mit Wasser versetzt, bis eine deutliche Phasentrennung der geschmolzenen Bistriazinylpolysulfane von den Lösungsmitteln eingetreten war.

Tabelle 1: Reaktionsdaten der Beispiele 5-9:

| Beisp. Nr. | Lös.-mittel | a in $Na_2S_a$ | Eduktstöchiometrien | | | Reaktionsbedingungen | |
|---|---|---|---|---|---|---|---|
| | | | Chlor-triazin*) (Mol) | $Na_2S_a$ (Mol) | Lös.-mittel (ml) | Zeit (h) | Temp. (°C) |
| 5 | Ethanol | 4,0 | 0,5 | 0,25 | 200 | 5,0 | 120**) |
| 6 | Glykol | 4,5 | 0,25 | 0,13 | 150 | 6,5 | 100-140 |
| 7 | n-Propanol + Wasser | 4,0 | 0,25 | 0,13 | 150+25 | 5,5 | 93- 98 |
| 8 | Glykol | 3,5 | 0,25 | 0,13 | 150 | 6,0 | 100-140 |
| 9 | Glykol | 5,0 | 0,25 | 0,13 | 200 | 4,5 | 100-140 |

*) gemeint ist 2-Ethylamino-4-diethylamino-6-chlortriazin

**) Autoklavenversuch

Tabelle 2: Produktdaten der Beispiel 5-9:

| Beispiel Nr. | Rohausbeute (%) | Anteil $S_8$ *) (%) | Elementenanalyse **) (%) C | H | N | S | Cl |
|---|---|---|---|---|---|---|---|
| 5 | 72,5 | 6,2 | 44,93 | 7,00 | 29,07 | 19,70 | 0,1 |
| 6 | 83,4 | - | 39,74 | 6,00 | 25,04 | 26,00 | 0,3 |
| 7 | 90,5 | - | 41,68 | 6,18 | 28,29 | 24,10 | 0,2 |
| 8 | 79,3 | - | 42,21 | 6,41 | 27,82 | 23,10 | 0,2 |
| 9 | 74,7 | 3,8 | 40,91 | 6,21 | 27,07 | 26,70 | 0,3 |

*) ermittelt durch Extraktion mit Ethanol

**) Berechnete Werte für:

| | C | H | N | S |
|---|---|---|---|---|
| a = 3,0: | 44,60 | 6,65 | 28,90 | 19,84 |
| a = 3,5: | 43,18 | 6,44 | 27,97 | 22,41 |
| a = 4,0: | 41,84 | 6,24 | 27,10 | 24,82 |
| a = 4,5: | 40,58 | 6,05 | 26,29 | 27,08 |
| a = 5,0: | 39,39 | 5,88 | 25,52 | 29,21 |

## Patentansprüche

1. Verfahren zur Herstellung von Mischungen schwefelhaltiger Triazinverbindungen der allgemeinen Formel

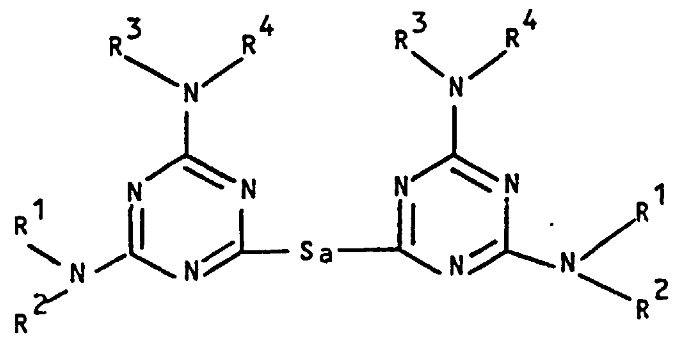

(I)

in welcher bedeuten:

| | |
|---|---|
| $R^1$, $R^2$ = | H, $R^2$ = Benzyl, |
| $R^2$, $R^3$, $R^4$ = | $C_1$-$C_8$-Alkyl, Allyl, $C_3$-$C_8$ Cycloalkyl,$C_1$-$C_8$-Alkyl, Allyl, Cycloalkyl, letzteres unsubstituiert oder mit 1-3 Methylgruppen substituiert, 2-Hydroxiethyl, 3-Hydroxipropyl, 2-Hydroxipropyl oder |
| $R^3$ und $R^4$ (zusammen): | $C_4$-$C_6$-Alkylen,-$(CH_2$-$CHX)_2$ Y mit X = H, $CH_3$, Y = O,S, $S_a$: Polysulfankette mit 2-10 S-Atomen (d.h. $2 \leq a \leq 10$), wobei die einzelnen Polysulfane in solchen Konzentrationen vorliegen, daß das statistische Mittel $\bar{a}$ ganze oder gebrochene Zahlenwerte von 2 bis 5 annimmt, dadurch gekennzeichnet, daß man eine Triazinverbindung der allgemeinen Formel |

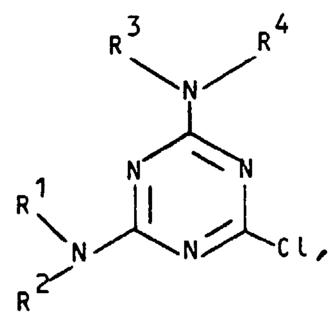

in der $R^1$, $R^2$,$R^3$, $R^4$ die oben angegebenen Bedeutungen besitzen,

mit einer in einem protischen, polaren Lösungsmittel, insbesondere Wasser oder deren Gemischen mit Wasser gelösten Verbindung der Formel

$$Me_2S_{\bar{a}} \qquad \text{(III)},$$

in der Me das Ammonium- oder ein Alkalikation bedeutet, und dem statistischen Mittelwert mit $2 \leq \bar{a} \leq 5$ entspricht, im Molverhältnis 2:1 bis 2:1,1 bei einer Temperatur von 80 bis 140°C umsetzt und aus dem anfallenden Reaktionsgemisch das Produkt in an sich bekannter Weise abtrennt.

**2.** Verfahren gemäß Anspruch 1, dadurch gekennzeichnet, daß man die Verbindung gemäß Formel (II) in geschmolzener Form in die wäßrige, auf 95 bis 100°C erhitzte Lösung der Verbindung gemäß Formel (III) eindosiert und während der bei dieser Temperatur ablaufenden Umsetzung eine wäßrige, alkalische Lösung in der Weise zusetzt, daß der pH-Wert des Reaktionsgemisches nicht unter 7,5 bis 9,0 sinkt.

**3.** Verfahren gemäß Anspruch 1, dadurch gekennzeichnet, daß man die Verbindungen gemäß den Formeln II und

III in dem jeweils verwendeten Lösungsmittel vorlegt und dann auf die Reaktionstemperatur aufheizt.

## Claims

**1.** Process for the production of mixtures of sulphur-containing triazine compounds of the general formula

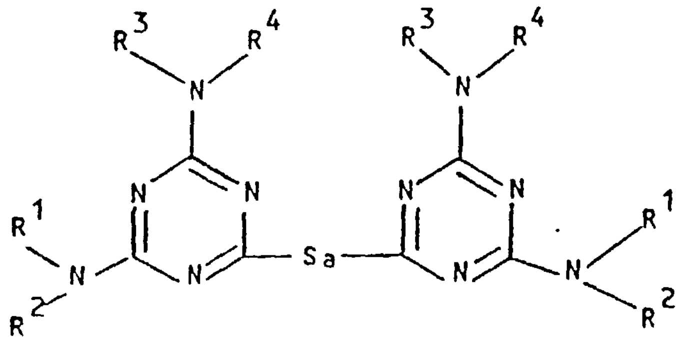

(I)

in which:

| | |
|---|---|
| $R^1$, $R^2$ = | H, $R^2$ = benzyl |
| $R^2$, $R^3$, $R^4$ = | $C_1$-$C_8$ - alkyl, allyl, $C_3$-$C_8$ cycloalkyl, the latter unsubstituted or substituted with 1-3 methyl groups, 2-hydroxy ethyl, 3-hydroxy propyl, 2-hydroxy propyl or |
| $R^3$ and $R^4$ (together): | $C_4$-$C_6$-alkylene, $-(CH_2\text{-}CHX)_2$ Y<br>with<br>X = H, $CH_3$, Y = O, S,<br>$S_a$: polysulphane chain having 2-10 S-atoms (i.e. $2 \leq a \leq 10$), wherein the individual polysulphanes are present in such concentrations that the statistical mean $\bar{a}$ assumes whole or fractional numerical values from 2 to 5, characterised in that a triazine compound of the general formula |

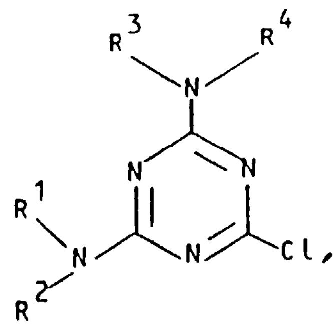

in which $R^1$, $R^2$, $R^3$, $R^4$ have the meanings given above, is reacted with a compound dissolved in a protic, polar solvent, in particular water, or mixtures of it with water, and of the formula

$$Me_2S_a \qquad \text{(III)},$$

in which Me means the ammonium- or an alkali cation, and a represents the statistical mean value $2 \leq a \leq 5$, in the mol ratio 2:1 to 2:1,1 at a temperature of 80 to 140°C, and from the resultant reaction mixture the product separates in a manner known per se.

**2.** Process acccording to claim 1, characterised in that the compound according to formula (II) is added in molten form into the aqueous solution of the compound according to formula (III) heated to 95 to 100°C, and during the reaction which occurs at this temperature an aqueous, alkaline solution is added in such a way that the pH value of the reaction mixture does not fall below 7,5 to 9,0.

**3.** Process according to claim 1, characterised in that the compounds according to formulae II and III are introduced in the respectively used solvent and then heated to the reaction temperature.

## Revendications

**1.** Procédé de préparation de mélanges de composés de triazine contenant du soufre de la formule générale :

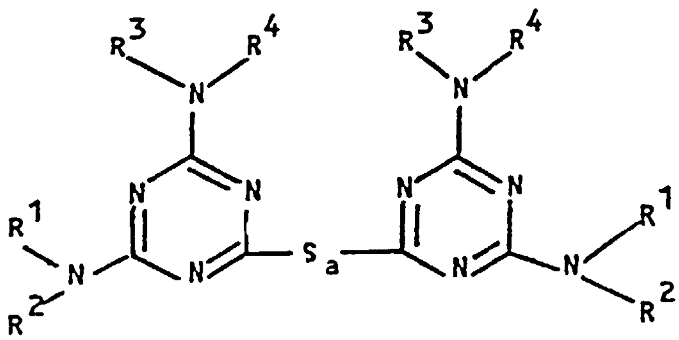

(I)

dans laquelle,

| | |
|---|---|
| $R^1$, $R^2$ = | H, $R^2$ = benzyle, |
| $R^2$, $R^3$, $R^4$ = | alkyle en $C_1$-$C_8$, allyle, cycloalkyle en $C_3$-$C_8$, ce dernier non substitué ou substitué avec des groupes méthyle en 1-3, 2-hydroxyéthyle, 3-hydroxypropyle, 2-hydroxypropyle ou |
| $R^3$ et $R^4$ (ensemble) signifient : | alkylène en $C_4$-$C_6$, $-(CH_2\text{-}CHX)_2Y$ avec X = H, $CH_3$, Y = O, S, |
| | $S_a$ : une chaîne polysulfane avec 2-10 atomes de S (c'est-à-dire $2 \leq a \leq 10$), tandis que les polysulfanes séparés sont présents dans des concentrations telles, que la moyenne statistique a prend des valeurs numériques entières ou fractionnaires de 2 à 5, |

caractérisé en ce qu'
on fait réagir un composé de triazine de la formule générale :

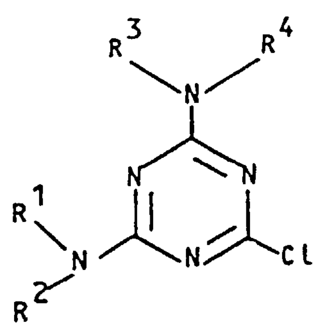

(II)

dans laquelle $R^1$, $R^2$, $R^3$, $R^4$ ont les significations indiquées plus haut,
avec un solvant protique, polaire, en particulier de l'eau, ou ses mélanges avec un composé dissout dans l'eau de la formule :

$$Me_2S_{\bar{a}} \qquad (III)$$

dans laquelle Me signifie l'ammonium ou une alcalinisation et a la valeur statistique moyenne avec $2 \leq \bar{a} \leq 5$, dans le rapport molaire 2:1 à 2:1,1 pour une température de 80 à 140°C et qu'on sépare le produit du mélange réactionnel résultant d'une manière connue en soi.

**2.** Procédé selon la revendication 1,
caractérisé en ce qu'
on introduit en dosant le composé selon la formule (II) sous forme fondue dans la solution aqueuse, chauffée de 95 jusqu'à 100°C du composé selon la formule (III) et on ajoute pendant la réaction qui se déroule à cette température une solution aqueuse alcaline de manière, que la valeur du pH du mélange réactionnel ne chute pas en dessous de 7,5 à 9,0.

**3.** Procédé selon la revendication 1,
caractérisé en ce qu'
on dépose les composés selon les formules II et III dans le solvant respectivement utilisé et qu'on chauffe alors à la température réactionnelle.